(19) Europäisches Patentamt
European Patent Office
Office européen des brevets

(11) Veröffentlichungsnummer: **0 020 969**
**A1**

(12) # EUROPÄISCHE PATENTANMELDUNG

(21) Anmeldenummer: **80102555.2**

(22) Anmeldetag: **09.05.80**

(51) Int. Cl.³: **C 07 C 101/54,** C 07 C 99/00 // C07D265/26

(30) Priorität: **20.06.79 DE 2924789**

(43) Veröffentlichungstag der Anmeldung: **07.01.81 Patentblatt 81/1**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Tonne, Peter, Dr., Triftbrunnenweg 63, D-6730 Neustadt 1 (DE)**
Erfinder: **Oeser, Heinz-Guenter, Dr., Friedrich-Burschell-Weg 19, D-6700 Ludwigshafen (DE)**
Erfinder: **Mangold, Dietrich, Dr., Hermann-Walker-Strasse 49, D-6903 Neckargemuend (DE)**

(54) **Verfahren zur Herstellung eines Gemischs von 3-Chloranthranilsäurealkylester und 6-Chloranthranilsäurealkylester.**

(57) Herstellung eines Gemischs von 3-Chloranthranilsäurealkylester und 6-Chloranthranilsäurealkylester in einem bestimmten Molverhältnis der Komponenten durch a) Umsetzung von 3-Chlorphthalsäureanhydrid mit Ammoniak, Alkalihydroxid und Alkalihypochlorit oder b) Umsetzung von 3-Chlorphthalsäureanhydrid zu 3-Chlorphthalamid mit anschließender Umsetzung mit Alkalihydroxid und Alkalihypochlorit und schließlich Veresterung des nach a) oder b) erhaltenen Gemisches von 5-Chlorisatosäureanhydrid und 8-Chlorisatosäureanhydrid mit Alkanolen.

Die nach dem Verfahren der Erfindung herstellbaren Verbindungen sind wertvolle Ausgangsstoffe für die Herstellung von Farbstoffen, Pflanzenschutzmitteln und Riechstoffen.

EP 0 020 969 A1

# Verfahren zur Herstellung eines Gemischs von 3-Chloranthranilsäurealkylester und 6-Chloranthranilsäurealkylester

Die Erfindung betrifft ein Verfahren zur Herstellung eines Gemischs von 3-Chloranthranilsäurealkylester und 6-Chloranthranilsäurealkylester in einem bestimmten Molverhältnis der Komponenten durch a) Umsetzung von 3-Chlorphthalsäureanhydrid mit Ammoniak, Alkalihydroxid und Alkalihypochlorit oder b) Umsetzung von 3-Chlorphthalsäureanhydrid zu 3-Chlorphthalimid mit anschließender Umsetzung mit Alkalihydroxid und Alkalihypochlorit und schließlich Veresterung des nach a) oder b) erhaltenen Gemisches von 5-Chlorisatosäureanhydrid und 8-Chlorisatosäureanhydrid mit Alkanolen.

Es ist aus Houben-Weyl, Methoden der Organischen Chemie, Band XI/1, Seiten 854 bis 862, zwar bekannt, daß man Carbonsäureamide durch Hofmann-Abbau mit Brom oder Chlor und Alkali in primäre Amine umsetzen kann. Ebenfalls ist aus Ullmanns Encyklopädie der technischen Chemie, Band 13, Seiten 723 bis 733 und DE-PS 127 138 bekannt, Phthalsäureanhydrid mit Ammoniak in Phthalimid zu verwandeln und aus diesem durch Umsetzung mit Hypohalogeniten in alkalischer Lösung Isatosäureanhydrid herzustellen. Bisher wurden diese Verfahrensweisen und die Veresterung so gebildeter Isatosäureanhydride für die Herstellung von 3-Chloranthranilsäurealkylestern nicht verwendet noch beschrieben. 3-Chloranthranilsäure wird hauptsächlich durch Umsetzung von o-Chloranilin mit Chloralhydrat und Hydroxylaminhydrochlorid zu o-Chlorisonitrosoacetanilid, anschließender Umsetzung des Anilids mit Schwefelsäure zu 7-Chlorisatin und dessen anschließender Umsetzung mit Wasserstoffperoxid hergestellt. Der Nachteil dieses Syntheseweges ist einmal in der Vielzahl der Stufen und zum anderen in der großen Menge an in der Stufe des o-Chlorisonitrosoacetanilids an-

WB/BL

fallenden umweltbelastenden Fremdsalzen zu sehen. Die Veresterung der 3-Chloranthranilsäure verläuft nur mit unbefriedigenden Ausbeuten.

Die Synthese der 6-Chloranthranilsäure nach dieser Arbeitsweise führt auf der Stufe des Isatins zu einem Isomerengemisch von 4- und 6-Chlorisatin (US-PS 3 184 462), aus dem das gewünschte 4-Chlorisatin erst nach einer aufwendigen Trennungsoperation in 45-prozentiger Ausbeute gewonnen werden kann.

Im Journal of The Chemical Society 1957, Seite 2 407, wird ein Verfahren zur Herstellung von 6-Chloranthranilsäure durch Oxidation von 2-Chlor-6-nitrotoluol mit Alkalipermanganat und anschließende Reduktion der 2-Chlor-6-nitrobenzoesäure mit Eisensulfat und wäßrigem Ammoniak beschrieben. Jedoch sind auch bei dieser Synthese die Ausbeuten (60 % für die Oxidation, 77 % für die Reduktion) unbefriedigend. Ebenfalls gibt es keine großtechnisch befriedigende Synthese des 2-Chlor-6-nitro-toluols, da sowohl die Chlorierung des o-Nitrotoluols (DE-PS 107 505) als auch die Nitrierung von o-Chlortoluol (Rec. Trav. Chim. Pay-Bas 32 (1913), Seite 283) diese Verbindung nur in Ausbeuten unter 30 % ergibt.

Es wurde nun gefunden, daß man ein Gemisch von 3-Chloranthranilsäurealkylester der Formel

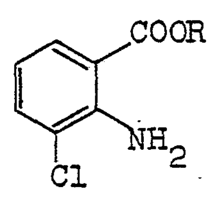 Ia,

und 6-Chloranthranilsäurealkylester der Formel

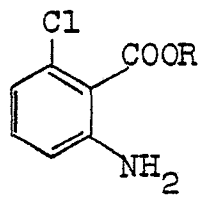 Ib,

worin R einen Alkylrest bedeutet, in einem Verhältnis von 2,9 bis 3,1 Mol des einen Esters zu einem Mol des anderen Esters, vorteilhaft herstellt, wenn man

a 1) 3-Chlorphthalsäureanhydrid mit Ammoniak und Alkalihypochlorit in Gegenwart von Alkalihydroxid bei einer Temperatur von 0 bis 100°C umsetzt und dann

a 2) das so gebildete Gemisch von 8-Chlorisatosäureanhydrid der Formel

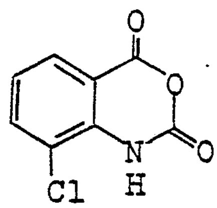 IIa,

und 5-Chlorisatosäureanhydrid der Formel

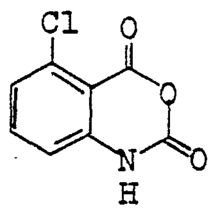 IIb,

mit einem Alkanol der Formel

ROH III,

worin R die vorgenannte Bedeutung besitzt, in Gegenwart eines Veresterungskatalysators verestert

oder

b 1) 3-Chlorphthalsäureanhydrid mit Ammoniak oder Formamid umsetzt und dann

b 2) das so gebildete 3-Chlorphthalimid mit Alkalihypochlorit in Gegenwart von Alkalihydroxid bei einer Temperatur von 0 bis 100°C umsetzt und dann

b 3) das so gebildete Gemisch von 8-Chlorisatosäureanhydrid der Formel

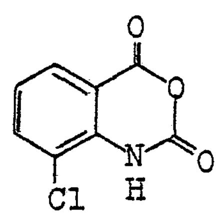

IIa

und 5-Chlorisatosäureanhydrid der Formel

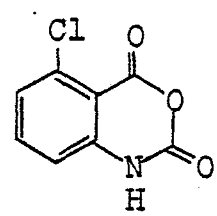

IIb,

mit einem Alkanol der Formel

ROH III,

worin R die vorgenannte Bedeutung besitzt, in Gegenwart eines Veresterungskatalysators verestert.

Die Umsetzung läßt sich für den Fall der Verwendung von Methanol und von Natriumhypochlorit im Falle der Verfahrensweise a (a 1 + a 2) durch die folgenden Formeln wiedergeben:

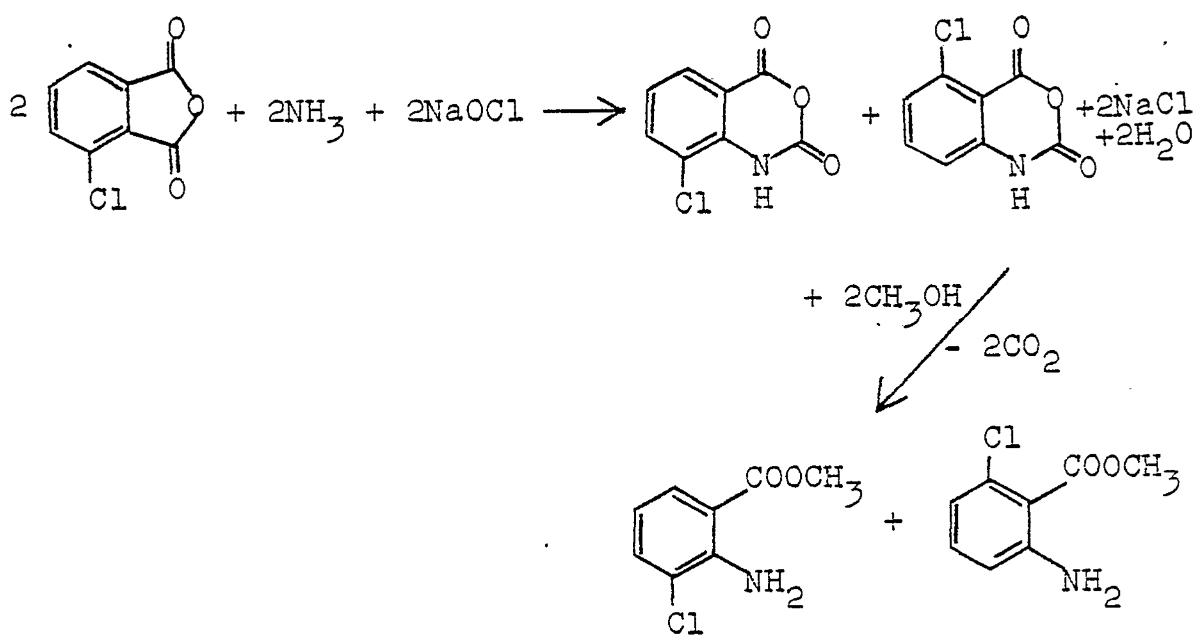


Die Umsetzung läßt sich für den Fall der Verwendung von Formamid, Methanol und von Natriumhypochlorit im Falle der Verfahrensweise b (b 1 + b 2 + b 3) durch die folgenden Formeln wiedergeben:

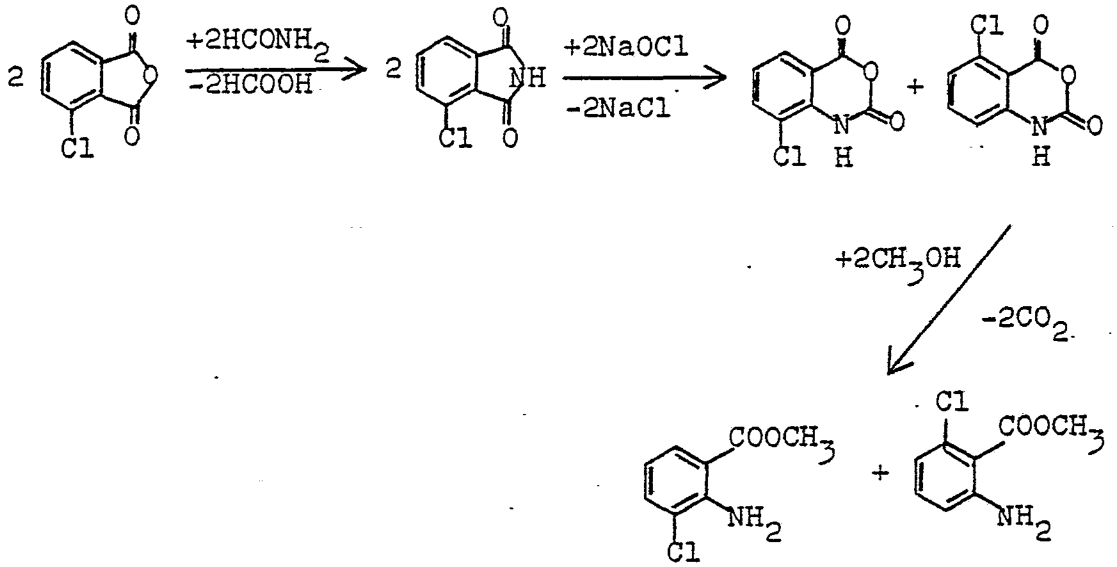


Im Vergleich zu den bekannten Verfahren liefert das Verfahren nach der Erfindung, ausgehend von dem leichter herstellbaren Ausgangsstoff 3-Chlorphthalsäureanhydrid bzw. 3-Chlorphthalimid, spezifische Gemische von 3-Chloranthranilsäurealkylester und 6-Chloranthranilsäurealkylester und die Einzelkomponenten der Gemische auf einfacherem und wirtschaftlicherem Wege in teilweise besserer Ausbeute und Reinheit. Nach der Verfahrensweise a) erhält man den 3-Chloranthranilsäurealkylester, insbesondere den Methylester, als Hauptprodukt und den 6-Chloranthranilsäurealkylester, insbesondere den Methylester, als Nebenprodukt, nach der Verfahrensweise b) erhält man den 6-Chloranthranilsäurealkylester, insbesondere den Methylester, als Hauptprodukt und den 3-Chloranthranilsäurealkylester, insbesondere den Methylester, als Nebenprodukt. In beiden Fällen liefert das Verfahren nach der Erfindung das Gemisch aber in einem defi-

nierten Verhältnis von Hauptprodukt zu Nebenprodukt. Alle diese vorteilhaften Ergebnisse des erfindungsgemäßen Verfahrens sind im Hinblick auf den Stand der Technik überraschend. Angesichts des reaktiven Chlorsubstituenten und der Reaktionskomponenten bei beiden Verfahrensweisen a) und b) hätte man eine erhebliche Ausbeuteverschlechterung bzw. die Bildung heterogener Gemische erwarten müssen. Ebenfalls ist es im Hinblick auf den Stand der Technik überraschend, daß ein wohl definiertes Isomerenverhältnis sowohl für das eine wie auch für das andere Isomer durch das erfindungsgemäße Verfahren zu erhalten ist, und somit sowohl der eine wie der andere Endstoff ohne wesentliche Betriebsumstellung in derselben Anlage hintereinander als Hauptprodukt hergestellt werden kann. Das erfindungsgemäße Verfahren ist daher ebenfalls für die Herstellung beider Endstoffe interessant, da diese aus den Gemischen durch einfache Destillation voneinander getrennt werden können. Die Arbeitsweise b) liefert daneben mit der Verwendung von Formamid die Möglichkeit, ein ammoniakfreies Phthalimid vorteilhaft in vorzüglichen Ausbeuten herzustellen, was für die Verwendung der Endstoffe in gegen Ammoniak empfindlichen Synthesen von Bedeutung ist.

Die Umsetzung wird zweckmäßig bei der Verfahrensweise a 1) und b 1) stöchiometrisch oder mit einem Überschuß von Ammoniak bzw. bei b 1) Formamid, vorteilhaft mit einem Molverhältnis von 1 bis 20, vorzugsweise 1 bis 5, insbesondere 1 bis 1,5 Mol Ammoniak oder Formamid zu 1 Mol Phthalsäureanhydrid, durchgeführt. Die Umsetzung der Stufe b 1) kann kontinuierlich oder diskontinuierlich, drucklos oder unter Druck, zweckmäßig bei einer Temperatur von 80°C bis 300°C, vorzugsweise von 100 bis 180°C, erfolgen. Man kann reines 3-Chlorphthalsäureanhydrid oder im großtechnischen Maßstab einfacher und wirtschaftlicher technisches, rohes Phthalsäureanhydrid verwenden, z.B. mit Konzentrationen von 90 bis

95 Gewichtsprozent reinem 3-Chlorphthalsäureanhydrid. Ebenfalls kann Ammoniak rein oder mit inerten Gasen, z.B. Stickstoff oder Kohlendioxid, vermischt sein. Die Reaktion der Stufe b 1) kann wie folgt durchgeführt werden: 3-Chlorphthalsäureanhydrid und Ammoniak bzw. Formamid werden bei der Reaktionstemperatur miteinander umgesetzt. Aus dem Reaktionsgemisch wird das 3-Chlorphthalimid dann in üblicher Weise, z.B. durch Vermischen mit Wasser und Filtration, abgetrennt.

Als weitere Ausgangsstoffe verwendet man Hypochlorite in wäßrigem Medium, in der Regel in Gestalt von entsprechenden wäßrigen, alkalischen Lösungen. Vorteilhaft gelangen wäßrige Lösungen oder Suspensionen von 1 bis 50 Gewichtsprozent 3-Chlorphthalsäureanhydrid (Verfahrensweise a 1) oder 3-Chlorphthalimid (Verfahrensweise b 2) zur Anwendung. Die wäßrigen Hypochloritlösungen enthalten im allgemeinen von 5 bis 15, vorzugsweise von 12 bis 14 Gewichtsprozent Hypochlorit und können zusätzlich von 0,2 bis 2,5 Mol, vorzugsweise 1 bis 2,1 Mol, Alkalihydroxid je Mol Hypochlorit enthalten. Bei dieser Menge von Alkalihydroxid wird das gegebenenfalls im Katalysator oder Hypochlorit enthaltene oder durch den Katalysator gebundene Alkali nicht eingerechnet. Im Ausgangsgemisch aller Ausgangsstoffe der Stufen a 1) oder b 2) kommen im allgemeinen Mengen von insgesamt 0,9 bis 1,5, vorzugsweise 0,95 bis 1,1 Mol Hypochlorit und zweckmäßig von insgesamt 0,9 bis 3 Mol, vorzugsweise 1 bis 2,1 Mol Alkalihydroxid (nicht eingerechnet das im Hypochlorit enthaltene Alkali), bezogen auf 1 Mol 3-Chlorphthalsäureanhydrid oder 3-Chlorphthalimid, in Frage. Enthält die wäßrige Hypochloritlösung kein freies Alkalihydroxid, so werden am Anfang oder im Laufe der Umsetzung zweckmäßig von 0,2 bis 2, vorzugsweise 1 bis 2 Mol Alkalihydroxid pro Mol Hypochlorit zugeführt. Bevorzugte Alkalihypochlorite sind das Natrium- oder das Kaliumsalz, bevorzugte Alkalihydroxide Natrium- oder Kaliumhydroxid. Bei der Verfahrensweise a 1) können auch zuerst Ammoniak, dann Alkalihydroxid, gegebenenfalls in Portionen und zuletzt das Hypochlorit zugegeben werden, wobei vorgenannte Mengen an Ammoniak und/oder Alkalihydroxid vorteilhaft sind.

Als Katalysatoren der Stufen a 1) oder b 2) kommen Brom, Jod und/oder Amide der Formel

$$X-\underset{|}{\overset{R^1}{N}}-R^2 \qquad IV,$$

worin $R^1$ eine Sulfonsäuregruppe, einen Sulfonatrest, eine Sulfonamidgruppe bezeichnet, $R^2$ ein Wasserstoffatom, einen aliphatischen Rest, ein Chloratom oder Bromatom bedeutet, X ein Chloratom, Bromatom oder Wasserstoffatom bezeichnet, $R^1$ und $R^2$ darüber hinaus auch zusammen mit dem benachbarten Stickstoffatom Glieder eines heterocyclischen Restes, der mindestens eine dem Stickstoffatom benachbarte Sulfongruppe oder Phosphonylgruppe der Formel $-\overset{O}{\overset{\|}{\underset{|}{P}}}-OR^3$, worin $R^3$ für ein Wasserstoffatom oder ein Alkaliatom steht, enthält, bezeichnen können, oder $R^1$ und $R^2$ zusammen auch den Rest $-\underset{O}{\underset{\|}{C}}-R^4-\underset{O}{\underset{\|}{C}}-$ bedeuten können, worin $R^4$ für einen Alkylenrest, den Rest $-\underset{R^5}{\underset{|}{N}}-\underset{O}{\underset{\|}{C}}-\underset{R^5}{\underset{|}{N}}-$ oder den Rest $-\underset{R^5}{\underset{|}{N}}-\underset{R^6\ R^6}{C}-$, $R^5$ für ein Wasserstoffatom, ein Chloratom oder Bromatom und $R^6$ für einen aliphatischen Rest stehen, im allgemeinen in einer Menge von 0,0001 bis 0,1, vorzugsweise von 0,001 bis 0,01 Mol Katalysator je Mol 3-Chlorphthalsäureanhydrid oder 3-Chlorphthalimid in Betracht. Anstelle der genannten Stoffe können auch Verbindungen, die unter den Reaktionsbedingungen solche Stoffe bilden, verwendet werden, z.B. Bromide und Jodide

anstelle von Brom oder Jod. Zweckmäßig wählt man wasserlösliche Halogenide. Diese Halogenide kommen vorteilhaft in Gestalt ihrer Erdalkali- und insbesondere ihrer Alkalisalze in Frage, z.B. Calciumbromid, Calciumjodid, Magnesiumbromid, Magnesiumjodid, Lithiumbromid, Lithiumjodid und insbesondere Natrium- und Kaliumbromid oder -jodid.

Bevorzugte Halogenamide IV sind solche, in deren Formel $R^1$ eine Sulfonsäuregruppe, einen Sulfonatrest, insbesondere einen Alkalisulfonatrest wie Natriumsulfonat oder Kaliumsulfonat, eine Sulfonamidgruppe bezeichnet, $R^2$ ein Chloratom, ein Bromatom, einen Alkylrest mit 1 bis 4 Kohlenstoffatomen oder insbesondere ein Wasserstoffatom bedeutet, X ein Bromatom, ein Chloratom oder zweckmäßig ein Wasserstoffatom bezeichnet, $R^1$ und $R^2$ darüber hinaus auch zusammen mit dem benachbarten Stickstoffatom Glieder eines heterocyclischen, 5- oder 6-gliedrigen Ringes, der mindestens eine dem Stickstoffatom benachbarte Sulfongruppe oder Phosphonylgruppe der Formel 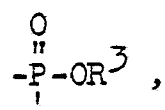 , worin $R^3$ für ein Wasserstoffatom oder ein Alkaliatom, insbesondere ein Natriumatom oder Kaliumatom steht, enthält, bezeichnen können oder $R^1$ und $R^2$ zusammen auch den Rest 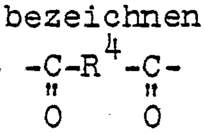 bedeuten können, worin $R^4$ für einen Alkylenrest mit 2 bis 4 Kohlenstoffatomen, den Rest 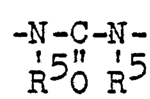 oder den Rest 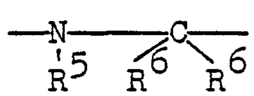 , $R^5$ für ein Wasserstoffatom, ein Chloratom oder Bromatom und $R^6$ für einen Alkylrest mit 1 bis 4 Kohlenstoffatomen, insbesondere die Methylgruppe, stehen. An den vorgenannten heterocyclischen Ring kann noch ein Phenylenkern anelliert sein. Vorteilhaft enthält der heterocyclische Rest 2 dem Stickstoffatom benachbarte Sulfon-oder Phosphongruppen oder zwei oder drei Sulfonamidogruppen oder Phosphonamidogruppen, insbesondere in demselben Ring bei mehrkernigen heterocyclischen Resten. Vorgenannte bevorzugte Reste können noch durch unter den Reaktionsbedingungen inerte Gruppen oder Atome, z.B. Chloratome, Bromatome, Alkylgruppen mit 1 bis 4 Kohlenstoffatomen, den Phenylkern substituierende Carboxyl- oder Carboxylatgruppen, substituiert sein.

Als Katalysatoren kommen z.B. in Betracht: Glutarimid, Adipinsäureimid, Succinimid; vorzugsweise Cyanursäure, 5,5-Dimethylhydantoin, Trisulfamid, N-Methyl-sulfaminsäure, Natriumtriimidometaphosphat; entsprechende Gemische vorgenannter Halogenamide IV; insbesondere sind Sulfaminsäure und ihre Salze, zweckmäßig Alkalisalze wie das Natrium- oder Kaliumsalz, und Sulfamid bevorzugt, gegebenenfalls im Gemisch mit vorgenannten Halogenamiden IV.

Gegebenenfalls können auch die in DE-OS 23 57 749 beschriebenen Polymerisationsinhibitoren als Katalysatoren der Schritte a 1) oder b 2), zweckmäßig nach der dort beschriebenen Verfahrensweise, angewendet werden.

Man setzt bei der Verfahrensweise a 1) oder b 2) bei einer Temperatur von 0 bis 100°C, vorzugsweise zwischen 10 und 85°C, drucklos oder unter Druck, kontinuierlich oder diskontinuierlich um. Die Reaktion a 1) oder b 2) kann wie folgt durchgeführt werden: Einem Gemisch von 3-Chlorphthalsäureanhydrid und Ammoniak (oder 3-Chlorphthalimid ohne Ammoniak), Katalysator, Alkalihydroxid und Wasser, gegebenenfalls zusammen mit einem Entschäumungsmittel, wird eine wäßrige Lösung des Hypohalogenits zugeführt und das Gemisch während 1 bis 4 000 Sekunden bei der Reaktionstemperatur gehalten. Zweckmäßig wird man das Alkali vor dem Hypochlorit mit dem Ausgangsgemisch vereinen. Nun wird

der Endstoff in üblicher Weise, z.B. durch Neutralisation des Reaktionsgemischs mit einer geeigneten Säure wie Schwefelsäure und Filtration, isoliert.

Man kann den Katalysator, zweckmäßig im Gemisch mit Wasser, auch dem Ausgangsgemisch getrennt oder zusammen mit dem Hypohalogenit zusetzen. In einer bevorzugten Ausführungsform, die gleichzeitig den besonders einfachen und vorteilhaften Betrieb von Anlagen nach dem erfindungsgemäßen Verfahren illustriert, wird zuerst aus 3-Chlorphthalsäureanhydrid, Ammoniak und gegebenenfalls Alkalihydroxid bei einer Temperatur von in der Regel 20 bis 80°C umgesetzt, dem so gebildeten Reaktionsgemisch direkt das Hypochlorit rasch, zweckmäßig in einem Guß, während 1 bis 100 Sekunden zugesetzt, und dann sofort mit Säure neutralisiert.

Bei der Stufe a 1) wird das Gemisch der Chlorisatosäureanhydride und damit in Stufe a 2) das Gemisch der Chloranthranilsäurealkylester in einem Verhältnis von 2,9 bis 3,1, vorzugsweise 2,95 bis 3,05 Mol 8-Chlorisatosäureanhydrid bzw. 3-Chloranthranilsäurealkylester je Mol 5-Chlorisatosäureanhydrid bzw. 6-Chloranthranilsäurealkylester, bei der Stufe b 2) wird das Gemisch der Chlorisatosäureanhydride und damit in Stufe b 3) das Gemisch der Chloranthranilsäurealkylester in einem Verhältnis von 2,9 bis 3,1, vorzugsweise 2,95 bis 3,05 Mol 5-Chlorisatosäureanhydrid bzw. 6-Chloranthranilsäurealkylester je Mol 8-Chlorisatosäureanhydrid bzw. 3-Chloranthranilsäurealkylester, hergestellt.

In der Stufe a 2) oder b 3) wird das Gemisch von 5-Chlorisatosäureanhydrid und 8-Chlorisatosäureanhydrid mit den Alkanolen III in stöchiometrischer Menge oder im Überschuß, vorzugsweise in einer Menge von 1 bis 50, insbesondere 1-30 Mol Alkanol III je Mol 5-Chlorisatosäureanhydrid

und 8-Chlorisatosäureanhydrid, umgesetzt. Bevorzugte Alkanole III und daher bevorzugte Chloranthranilsäurealkylester Ia und Ib sind solche, in deren Formeln R einen Alkylrest mit 1 bis 18, vorteilhaft 1 bis 8, insbesondere 1 bis 4 Kohlenstoffatomen bedeutet. Geeignete Alkanole sind z.B. Äthanol, n-Propanol, Isopropanol, Butanol, Isobutanol, sek.-Butanol, tert.-Butanol, Pentanol, 2,2-Dimethylpropanol, 2-Äthylhexanol, Hexanol, Heptanol, Octanol, 2-Äthyloctanol und bevorzugt Methanol. Die Reaktion wird in der Regel bei einer Temperatur zwischen 10 und 170°C, vorzugsweise zwischen 20 und 90°C, drucklos oder unter Druck, kontinuierlich oder diskontinuierlich durchgeführt. Als Katalysatoren werden zweckmäßig Säuren wie Phosphorsäure, aromatische Sulfonsäuren wie p-Toluolsulfonsäure oder insbesondere Chlorwasserstoff und Schwefelsäure zugesetzt; vorteilhaft sind insbesondere 0,5 bis 15, insbesondere 3 bis 10 Gewichtsprozent Säure, bezogen auf Ausgangsstoffe IIa und IIb. Gegebenenfalls können Stoffe, die das gebildete Wasser binden, mitverwendet werden, z.B. wasserfreie Salze wie Kupfersulfat, Eisensulfat. Ebenfalls kommen Veresterungskatalysatoren wie Säurechloride, z.B. Thionylchlorid, Chlorsulfonsäure, Ansolvosäuren wie Bortrifluorid, oder spezielle Veresterungsoperationen, z.B. Veresterung unter azeotroper Destillation mit z.B. Benzol oder Toluol, in Frage.

Bevorzugt verwendet man als Katalysatoren auch Basen, in der Regel tertiäre Amine, z.B. Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diäthylanilin, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin. Die Umsetzung wird in Gegenwart der basischen Verbindung vorteilhaft in einer Menge von 0,01 bis 0,5, vorzugsweise von 0,05 bis 0,35 Äquivalenten basischer Verbindung, bezogen auf ein Mol Ausgangsstoff IIa und IIb, durchgeführt. Ebenfalls können als Katalysatoren Alkalihydroxide oder Alkalicarbonate, z.B. nach der im J. Org. Chem., Band 24 (1959), Seiten 1 214 bis 1 219 beschriebenen Arbeitsweise, verwendet werden.

Die Umsetzung der Stufe a 2) oder b 3) kann wie folgt durchgeführt werden: Ein Gemisch der Ausgangsstoffe IIa, IIb und III, Veresterungskatalysator, Säure bzw. Base, wird bei vorgenannter Temperatur unter Rühren während 1 bis 12 Stunden gehalten. Nun werden die Ester I in üblicher Weise, z.B. durch fraationierte Destillation, isoliert. Bezüglich der Einzelheiten der Veresterungsreaktion wird auf Houben-Weyl, Methoden der Organischen Chemie, Band 8, Seiten 516 bis 549, verwiesen.

Die nach dem Verfahren der Erfindung herstellbaren Verbindungen Ia und Ib sind wertvolle Ausgangsstoffe für die Herstellung von Farbstoffen, Pflanzenschutzmitteln und Riechstoffen. Bezüglich der Verwendung wird auf vorgenannte Veröffentlichungen verwiesen.

Die in den folgenden Beispielen angeführten Teile bedeuten Gewichtsteile. Sie verhalten sich zu den Volumenteilen wie Kilogramm zu Liter.

Beispiel 1

a 1) In 70 000 Volumenteilen Wasser und 6 480 Volumenteilen Ammoniak (21-gewichtsprozentig) werden bei 30°C 6 800 Teile 3-Chlorphthalsäureanhydrid eingerührt, wobei der pH-Wert sich auf 5,3 einstellt. Anschließend

wird das Gemisch mit 50-gewichtsprozentiger Natronlauge auf pH 10 eingestellt und dann mit 4 300 Volumenteilen 50-gewichtsprozentiger Natronlauge und weiteren 6 800 Teilen 3-Chlorphthalsäureanhydrid versetzt. Man rührt das Gemisch 4 Stunden nach und fügt dann 250 Teile Triisobutylphosphat und 150 Teile Sulfaminsäure und weitere 100 Teile 3-Chlorphthalsäureanhydrid hinzu. Der pH-Wert der Lösung wird mit Natronlauge (50 Gewichtsprozent) auf pH 10 eingestellt und das Gemisch nun auf 3°C abgekühlt. Zu dieser Lösung wird unter starkem Rühren bei 5°C innerhalb von 45 Sekunden eine Lösung von 60 000 Volumenteilen Wasser und 32 000 Volumenteilen 13,4-gewichtsprozentiger Natriumhypochloritlösung gegeben. Der pH-Wert steigt auf 12,5 an. Anschließend wird das Gemisch mit 25-gewichtsprozentiger Schwefelsäure auf pH 7 eingestellt, dann auf 30°C erhitzt und durch weitere Zugabe von 25-gewichtsprozentiger Schwefelsäure bei pH 7 gehalten. 2,5 Stunden nach der ersten Säurezugabe wird der ausgefallene Niederschlag abgesaugt, mit Wasser gewaschen und im Vakuum getrocknet. Man erhält 11 045 Teile (77 % der Theorie) eines Gemisches von 8-Chlorisatosäureanhydrid und 5-Chlorisatosäureanhydrid im Verhältnis 3 : 1 vom Fp 210 bis 218°C.

a 2) Eine Suspension von 26 700 Teilen eines Gemisches von 8-Chlor- und 5-Chlorisatosäureanhydrid (3 : 1) und 150 000 Volumenteilen Methanol wird zum Rückfluß erhitzt. Nun werden innerhalb von 2 Stunden 5 000 Volumenteile Triäthylamin zugegeben, wobei eine gleichmäßige Kohlendioxidentwicklung stattfindet. Anschließend wird das Gemisch noch eine Stunde am Rückfluß erhitzt, der Alkohol im Vakuum abdestilliert und der Rückstand im Vakuum fraktioniert destilliert. Man erhält 15 220 Teile (60,7 % der Theorie) 3-Chloranthranilsäuremethylester vom Kp (0,3 mbar) 100°C und 5 066 Teile (20,2 % der Theorie) 6-Chloranthranilsäuremethylester vom Kp (0,3 mbar) 117°C.

Beispiel 2

b 1) 300 Teile 3-Chlorphthalsäureanhydrid werden in 1 200 Volumenteilen Formamid 2 Stunden bei 140°C gerührt. Anschließend läßt man das Gemisch auf 100°C abkühlen und gießt die Lösung dann zu 5 000 Volumenteilen Wasser. Der ausgefallene Niederschlag wird abgesaugt, mit Wasser gewaschen und im Vakuum getrocknet. Man erhält 275 Teile (92,2 % der Theorie) 3-Chlorphthalimid vom Fp 236 bis 238°C.

b 2) Eine 3-Chlorphthalimidlösung (bestehend aus 182 Teilen 3-Chlorphthalimid, 2 500 Volumenteilen Wasser und 82 Teilen 50-gewichtsprozentiger Natronlauge) wird mit einem Teil Sulfaminsäure und 3 Volumenteilen Triisobutylphosphat versetzt. Hierzu wird bei 5°C unter starkem Rühren in einem Guß eine Lösung von 550 Teilen 13,4-gewichtsprozentiger Natriumhypochloritlösung in 800 Volumenteilen Wasser gegeben. Nach 10 Sekunden ab Beginn der Hypochloritzugabe wird das Gemisch durch Zugabe von 25-gewichtsprozentiger Schwefelsäure auf pH 7 eingestellt und durch weitere Säurezugabe bei pH 7 gehalten. 1,5 Stunden nach der ersten Säurezugabe wird der ausgefallene Niederschlag abgesaugt, mit Wasser gewaschen und im Vakuum bei 60°C getrocknet. Man erhält 174 Gewichtsteile (88 % der Theorie) eines Gemisches von 8-Chlorisatosäureanhydrid und 5-Chlorisatosäureanhydrid im Verhältnis 1 : 3 vom Fp 244 bis 246°C (Zersetzung).

b 3) Eine Suspension von 500 Teilen eines Gemisches von 8-Chlor- und 5-Chlorisatosäureanhydrid (1 : 3) und 2 000 Volumenteilen Methanol wird analog Beispiel 1 a 2) mit 25 Volumenteilen Triäthylamin zum Chloranthranilsäuremethylestergemisch umgesetzt. Man erhält 96 Teile (20,4 % der Theorie) 3-Chloranthranilsäuremethylester vom Kp (0,3 mbar) 100°C und 288 Teile (61,3 % der Theorie) 6-Chloranthranilsäuremethylester vom Kp (0,3 mbar) 117°C.

Patentanspruch

Verfahren zur Herstellung eines Gemischs von 3-Chloranthranilsäurealkylester der Formel

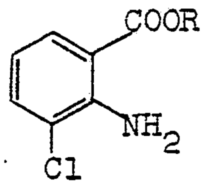

Ia,

und 6-Chloranthranilsäurealkylester der Formel

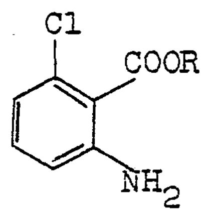

Ib,

worin R einen Alkylrest bedeutet, in einem Verhältnis von 2,9 bis 3,1 Mol des einen Esters zu einem Mol des anderen Esters, dadurch gekennzeichnet, daß man

a1) 3-Chlorphthalsäureanhydrid mit Ammoniak und Alkalihypochlorit in Gegenwart von Alkalihydroxid bei einer Temperatur von 0 bis 100°C umsetzt und dann

a2) das so gebildete Gemisch von 8-Chlorisatosäureanhydrid der Formel

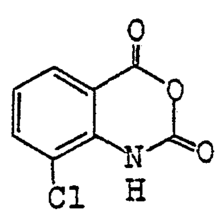

IIa,

und 5-Chlorisatosäureanhydrid der Formel

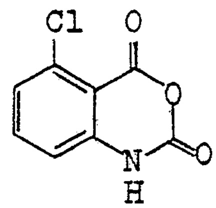 IIb,

mit einem Alkanol der Formel

ROH III,

worin R die vorgenannte Bedeutung besitzt, in Gegenwart eines Veresterungskatalysators verestert

oder

b1) 3-Chlorphthalsäureanhydrid mit Ammoniak oder Formamid umsetzt und dann

b2) das so gebildete 3-Chlorphthalimid mit Alkalihypochlorit in Gegenwart von Alkalihydroxid bei einer Temperatur von 0 bis 100°C umsetzt und dann

b3) das so gebildete Gemisch von 8-Chlorisatosäureanhydrid der Formel

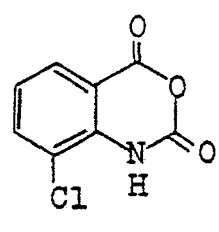 IIa,

und 5-Chlorisatosäureanhydrid der Formel

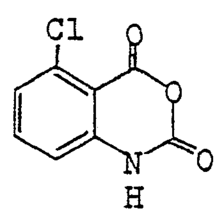 IIb,

mit einem Alkanol der Formel

ROH III,

worin R die vorgenannte Bedeutung besitzt, in Gegenwart eines Veresterungskatalysators verestert.

Europäisches Patentamt

# EUROPÄISCHER RECHERCHENBERICHT

0020969
Nummer der Anmeldung
EP 80 10 2555

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CHEMICAL ABSTRACTS, Band 47, No.5, Zusammenfassung 2143b, 10-03-1953, Columbus, Ohio, US, M.B. CHAUDHARI et al.: "Hofmann reaction of 3-halophthalimides" & J. UNIV. BOMBAY, Sect. A 19, Pt. 3 | 1 |
| | DE - C - 139 218 (BASLER CHEMISCHE FABRIK)<br>* Beispiele * | 1 |
| D | EUGEN MÜLLER: "Methoden der Organischen Chemie", Houben-Weyl, vierte Auflage, Band XI/1, 1957, Georg Thieme Verlag, Stuttgart, DE, "Stickstoffverbindungen II", Seiten 854-857<br>* Seiten 856,857 * | 1 |
| D | DE - C - 127 138 (FARBWERKE VORM. MEISTER LUCIUS & BRUNING)<br>* Beispiele * | 1 |
| | US - A - 3 123 631 (R.P. STAIGER)<br>* Spalte 1, Zeile 33 bis Spalte 2, Zeile 40; Beispiele * | 1 |
| | GB - A - 1 549 297 (IMPERIAL CHEMICAL INDUSTRIES)<br>* Seite 1, Zeilen 13-61 * | 1 |

KLASSIFIKATION DER ANMELDUNG (Int. Cl.[3])

C 07 C 101/54
99/00
C 07 D 265/26

RECHERCHIERTE SACHGEBIETE (Int. Cl.[3])

C 07 C 101/54
99/00
C 07 D 265/26

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 16-09-1980 | PAUWELS |